# EUROPEAN PATENT APPLICATION

(11) **EP 3 922 991 A1**
(43) Date of publication of application: **15.12.2021**
(21) Application number: 20179317.1
(22) Date of filing: 10.06.2020
(51) Int. Cl.: G01N 33/543

(54) **DISPERSION USING A MOVING MAGNET**

(71) Applicant: PreOmics GmbH, 82152 Planegg/Martinsried (DE)
(72) Inventor: Kulak, Nils A., 80686 München (DE); Hartinger, Katrin, 81249 München (DE); Käsemann, Martin, 40789 Monheim (DE); Johansson, Sebastian, 82284 Grafrath (DE); Wächter, Jasmin, 82284 Grafrath (DE)
(74) Representative: Dentons Patent Solutions Rechtsanwaltsgesellschaft mbH

(57) **Abstract**

This invention relates to a method of dispersing magnetic particles, said method comprising or consisting of: (a) in a vessel, combining at least one permanent magnet and said magnetic particles in a liquid phase; and (b) triggering a fluctuating or oscillating motion of said permanent magnet using a magnetic field; thereby dispersing said particles.

## Description

The present invention relates to a method of dispersing magnetic particles, said method comprising or consisting of: (a) in a vessel, combining at least one permanent magnet and said magnetic particles in a liquid phase; and (b) triggering a fluctuating or oscillating motion of said permanent magnet using a magnetic field; thereby dispersing said particles.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Magnetic separation technologies are well established and commonly applied, e.g. in chemical, bioscience and diagnostic applications. The methods rely on selectively dispersing or magnetizing functionalized nano- to micrometer sized para- or ferromagnetic particles in a suspension setting. While particles are dispersed, equilibration, binding, reaction, washing or elution conditions can be applied. During the magnetized phase, particles and supernatant can be separated.

Advantages of these methods include unrestricted volumes, the implementation option as a direct batch method, and applicability even in the presence of crude impurities such as cellular remnants. The simple addition and removal of solid materials, independent of target material is, e.g. especially suitable for DNA enrichment where target DNA molecules can be extremely large. Furthermore, little to no hands-on work and no time-consuming centrifugation steps are required. The easy prevention of potential cross-contamination and amenability to automation make magnetic separation techniques ideal for low- to high-throughput applications.

Independent of the application, a magnetic separation of the particles has to be performed, typically repeatedly. Conventionally, most manual and the majority of automated methods employ strong permanent magnets which are temporarily brought into close proximity of an external face of the vessel (commonly polymer reaction tubes in the range of 5 µL to 50 mL) in which the magnetic particles are held. The particles are magnetized and collect in proximity of the point where the magnetic field is strongest. This moves the magnetized particles out of dispersion into an aggregation or collection. If very small particles are used, generally stronger magnets need to be employed and/or more time has to elapse until gathering of the particles is complete, thereby providing for a substantially complete recovery of particles. This aggregation step depends on magnetic forces attracting the particles to the magnetic field. To bring the particles back in dispersion, the magnet is moved away from the vessel (and thereby from the particles), and the particles can again be dispersed by mixing such as up-and-down pipetting or shaking.

Non-permanent magnets can also be employed as magnetic separators. Notably, PerkinElmer's Chemagic platforms (PerkinElmer Chemagen Technologie GmbH) use a system of transiently magnetized rods. Here, electromagnets are used for handling larger volumes and are directly submerged into the solution containing the magnetic particles (Chemagic Magnetic Separation Module I (<10 ml); Magtration^{®} System 8 1□×□(7 ml)).

All these systems share a common feature in that they use physical movement to either introduce or to move and remove the source for magnetization towards and away from of the magnetic particles, respectively. The mentioned Chemagic platform, while using a different approach for magnetic separation, requires introduction of a rod into the sample which may cause cross-contamination. Moreover, the requirement for separate mixing and dispersion procedures with different associated technical means is feature shared with those prior art methods discussed further above which employ a magnet which is entirely external to the reaction vessel.

In view of the shortcomings of the prior art, the technical problem underlying the present invention can be seen in the provision of improved means and methods of handling magnetic particles. Related thereto, and depending on the specific envisaged application, the technical problem can also be seen as the provision of improved means and methods of separation or synthesis, more specifically of separation or synthesis by magnetic means.

This problem is solved by the subject-matter of the claims.

Accordingly, in a first aspect, the present invention provides a method of dispersing magnetic particles, said method comprising or consisting of: (a) in a vessel, combining at least one permanent magnet and said magnetic particles in a liquid phase; and (b) triggering a fluctuating or oscillating motion of said permanent magnet using a magnetic field; thereby dispersing said particles.

Magnetic particles in a liquid phase generally settle down owing to gravity. This is deleterious to their interaction with any constituents of said liquid phase. For that reason, there is need for keeping them in suspension to achieve efficient surface contact with the entire liquid phase. This is referred to as "dispersing" and is classically achieved using mechanical means such as shaking, vortexing or up- and down-pipetting. In accordance with this invention, dispersing is achieved by a fluctuating or oscillating motion of said permanent magnet.

Magnetic particles (often referred to as magnetic microparticles; magnetic nanoparticles; magnetic beads; magnetic microspheres; paramagnetic, ferrimagnetic or ferromagnetic beads) are particles which respond to a magnetic field. Generally, they are paramagnetic or ferromagnetic.

A multitude of functionalized magnetic particles is known in the art. Functionalization may be confined to the surface of the particles or may, for example in case of porous particles, extend to the interior surface inside such pores.

Functionalization enable magnetic particles to interact with material in their proximity with or without triggering a chemical modification of said material. In other words, there may be a non-covalent interaction between e.g. an analyte of interest and a moiety on a magnetic particle. This is also referred to as "binding" in the following. On the other hand, magnetic particles may be equipped with moieties which are capable of triggering a chemical reaction which leads to the formation of a new chemical compound. This is referred to as the formation of a "product" or of an "adduct" in this specification.

Preferred functionalizations, i.e., preferred moieties bound to magnetic particles, are subject of preferred embodiments disclosed further below.

In terms of size, magnetic particles preferably have a diameter between 1 nm and 1 mm, or between 100 nm and 60 µm. Preferably, for a given choice of magnetic particles, their size distribution is narrow. Also, preference is given to particles which are chemically inert. This may be achieved by a coating. Furthermore, physical stability has been an issue in the past. As such, preference is given to particles which do not break apart under normal conditions of use. The magnetic material comprised in magnetic particles may also be embedded in a matrix such as silica or a polymer. This is also a means of conferring chemical and/or physical stability.

Generally, a large number of magnetic particles is used. The actual numbers depend on the application chosen and its scale. Exemplary values would be between 100 and 100000 particles per vessel, such as between 1000 and 10000.

Prior to use, magnetic particles are preferably equilibrated. In case magnetic particles provided by one of the manufacturers given further below are used, equilibration may follow the guidelines given by the manufacturer. Generally speaking, equilibration involves suspending the particles in an aqueous solution such as a buffer.

Said combining may occur concomitantly or in any order.

Said liquid phase is not particularly limited. Implementations include a sample or mixture comprising one or more compounds or analytes of interest. Analytes may belong to classes. For example, a proteome would be a collection of molecules which share the feature of being proteinaceous in nature. The term "liquid phase" embraces solutions and suspensions.

Samples may contain further material in addition to the molecules of interest. Such further material may be contaminants the removal of which is desirable. Liquid phases may also be pure on the other hand: they may be solutions of a compound of interest which do not contain further constituents in addition to the compound of interest and a solvent.

A key feature of the liquid phase is that it provides an environment where interaction between said magnetic particles, more specifically the moieties on the surface of said particles, with any analyte or compound of interest in said liquid phase may occur.

Magnetic particles are in widespread use, applications ranging from purification of analytes to solid phase synthesis. In order to optimize yield of these processes, the reaction mixture comprising the analyte or the starting material together with said particles are mixed, which conventionally is generally done by pipetting up and down, by shaking, or by vortexing.

The present inventors unexpectedly found that adding at least one permanent magnet to the reaction mixture and triggering motion of said permanent magnet by means of a magnetic field, said magnetic field being generated by a further magnet, said further magnet being located outside the vessel holding the reaction mixture, is a means of mixing which is both effective and convenient and confers a number of distinct advantages.

Of note, also the prior art approaches use a magnet to handle magnetic particles, namely, to collect them in one or a few defined places inside the vessel such that any remaining liquid comprising unbound matter can be removed. Yet, the prior art approaches do not use the magnet for mixing. Rather, during the mixing phase, the magnet is absent.

As such, the contribution the present invention makes to the art is the use of an element, namely a magnet inside a vessel holding a reaction mixture with magnetic particles, not only for collecting the particles, but also and in particular for mixing the reaction mixture.

Using a magnet for mixing also renders the mixing process as such more efficient as compared to pipetting up and down or shaking: the permanent magnet, owing to the fluctuating or oscillating motion it performs, renders the flow (in a rheological sense) of the liquid reaction mixture more turbulent. Furthermore, the attractive forces between magnetic particles and said permanent magnet lead to a motion of the magnetic particle which is not in phase with the movement of the liquid. Of note, the magnetic force exerted by the external magnet is alien to the prior art mixing processes as are the mentioned advantages entailed thereby.

As demonstrated in the Examples enclosed herewith, where the number of peptides identified in a subsequent analysis by mass spectrometry is used to assess performance of the method, the invention performs at least as good as conventional magnetic particle handling and at the same confers the advantages discussed above.

The above disclosed vessel is not particularly limited.

Useful vessels include those which are generally used in the field of molecular biology and in vitro diagnostics. Such vessels are generally free or substantially free of contaminants, chemically inert, and/or have surfaces with low binding capacity.

Important in the context of the present invention is that vessels have at least one wall which does not shield magnetic fields. Preferably, the entire vessel is made of a material which does not shield magnetic fields. Suitable materials include plastic, polymers such as polypropylene, glass, and ceramics. Keeping the requirement of magnetic permeability in mind, also metals may be used.

Exemplary and preferred vessels are those which are configured to hold a volume of 5 µL to 1 L, preferably between 10 µL to 50 mL, more preferably configured to hold volumes of any of 30 µL, 40 µL, 100 µL, 150 µL, 200 µL, 250 µL, 500 µL, 1 mL, 1.5 mL, 2 mL, 5 mL, 15 mL and 50 mL.

Vessels may be arranged in arrays, such as the common formats (such as one- or two-dimensional arrays with e.g. 6, 24, 96, 384 or 1546 wells).

Vessels may also be implemented as microfluidic devices, i.e., miniaturized devices comprising one or more channels with openings, optionally with widenings or containers and/or valves.

The term "vessel" furthermore embraces vessels with a closed bottom, vessels with a lid, vessels with a closed bottom and a lid, entirely closed or sealed vessels, tubular elements, and elements with at least two openings allowing for continuous flow of a liquid phase through such an element. Also, in such a flow-through vessel or flow-through reactor, said at least one permanent magnet and its motion are controlled by the magnetic field.

Preferably said magnetic field is generated by a magnet which external to said vessel. The external magnet may be implemented in various ways; for further details see below. Generally speaking, what is key is that the magnetic field is capable of triggering the fluctuating or oscillating motion of the permanent magnet located inside the vessel. This can be achieved by a magnetic field which changes over time at the site of the vessel. Such change over time can be effected by a movement of the element which generates the field in space. Alternatively, or in addition, this may be done by changing the field-generating parameters over time, e.g., by changing the electric current over time which flows through an electric conductor such as a coil.

Accordingly, said magnetic field may be generated by an electromagnet, wherein the current flowing through said electromagnet changes over time such that the at least one permanent magnet under the influence of said field performs the fluctuating or oscillating motion required, e.g. for mixing.

It may also be generated by a permanent magnet, said permanent magnet under such circumstances being a further permanent magnet, given that at least one permanent magnet is inside the vessel. This - external - permanent magnet may perform a movement in three-dimensional space, the consequence being that the magnetic field generated by said external permanent magnet at the site of the vessel changes over time - again, for the purpose of generating said fluctuating or oscillating motion.

A permanent magnet is a piece of ferri- or ferromagnetic material.

Sizes of single magnets may vary widely. As far as the permanent magnet(s) inside the vessel are concerned, they may be appropriately chosen in dependency of the dimension of the vessel or, equivalently, reactor to be used.

In terms of relative size, it is preferred that the largest dimension of the permanent magnet fits through the smallest passage or cross-section in said reactor or vessel. Smaller than the smallest dimension preferably means 2/3, ½, 1/3, ¼, or 10% of said smallest dimension or cross-section of said vessel. Such setup generally provides for free or substantially free motion. Preferably, free or substantially free motion occurs around or along at least two, at least three, at least four, at least five or preferably all six axes of translational and rotational motion, and wherein preferably said free or substantially free motion includes translation along at least two axes.

To explain, for a point-like object, there are three degrees of motional freedom, i.e., translation in three independent directions spanning the three-dimensional space. For an extended object, there are three further degrees of freedom which can be defined in terms of three independent axes of rotation.

For the sake of completeness, exemplary values of the size of a useful permanent magnet are given here to be between 0.1 mm and 10 cm such as between 0.2 mm and 2 cm, including any of the following values and ranges defined thereby: 0.3 cm, 0.4 cm, 0.5 cm, 0.6 cm, 0.7 cm, 0.8 cm, 0.9 cm, and 1 cm. The same preferred sizes and size ranges apply to any permanent magnet considered herein, also to implementations where use is made of a plurality of magnets. Generally, these lengths refer to the largest extension of said permanent magnet.

Alternatively, a plurality of permanent magnets such as those defined above may be used. Exemplary, but non-limiting numbers are in the one-digit and two-digit range such as 2, 3, 4, 5, 6, 7, 8, 9, and 10. Also more than 10 such as 20, 30, 40, or 50 magnets may be used. These numbers refer to one vessel, also in case arrangements of vessels (such as microtiter plates) are used.

It is understood that the number of permanent magnets, to the extent it is specified, is limiting. In other words, while the method of the first aspect may comprise other measures, such possibility does not extend to the option of more permanent magnets being present than expressly specified.

The magnets of such a plurality of magnets may be identical or different from each other.

The same applies to magnets in general, to the extent a plurality thereof is to be used. To give a specific example, a single magnet may be combined with a single ferro- or ferrimagnetic bead. This provides for more rigorous motion of the magnet while the parameters controlling the magnetic field are left unchanged; see also further below.

A further means of achieving more rigorous motion is the addition of one or more such 2, 3, 4, 5, 6, 7, 8, 9 or 10 beads made of non-magnetizable material such as non-magnetic metals. Particularly preferred is one such bead and/or such bead(s) being of the same size or comparable to the size of the permanent magnet.

In terms of shape of said permanent magnet, there are no particular limitations, wherein preference is given to those shapes which do not negatively interfere with the free motion of the magnet. Exemplary shapes include sticks, bars, rods, rods with rounded ends, cubes, cuboids, prisms, spheres, elongate and oblate ellipsoids, disks, tetrahedrons, octahedrons, dodecahedrons, and icosahedrons.

In a further preferred embodiment, (a) said permanent magnet comprises or consists of ferromagnetic material or ferrimagnetic material; and/or (b) said magnet and/or said particles are coated, preferably with a coating selected from (i) a coating conferring chemical stability; (ii) a coating conferring mechanical stability or hardness; (iii) a coating with a catalyst; (iv) a coating with a nucleic acid such as a probe and/or primer; (v) a coating with a chelating agent such as IMAC, TiO₂ and ZrO₂; (vi) a coating with a chromatographic material, preferably selected from (1) reversed phase groups such as C18, C8, Benzene; (2) HILIC groups such as hydroxyl groups; (3) cation ionexchange groups such as sulfonic acid, phosphoric acid, carboxylic acid; (4) anion-exchange groups such as primary, secondary, tertiary and quaternary amino groups; and (5) any combination of any one of (1) to (4); (vii) a coating with ligand-binding proteins and/or their cognate ligands; preferably selected from globulins, particularly immunoglobulins; antigens, preferably antigens capable of binding to immunoglobulins; streptavidin, biotin; Protein A, Protein G; annexin V, phospholipids; enzymes such as oxidoreductases, transferases, ligases such as polymerases, hydrolases such as proteases, peptidases, nucleases, saccharidases, lipases, lyases, and isomerases; lipids; and (viii) a combination of any one of (i) to (vii).

Suitable materials for said permanent magnets include the following elements and their alloys: neodymium-iron, neodymium-iron-boron (e.g. Nd₂Fe₁₄B), cobalt, gadolinium, terbium, dysprosium, iron, nickel, iron oxides, manganese-bismuth, manganese-antimony, manganese-arsenic, yttrium-iron oxides, chromium oxides, europium oxides, and samarium-cobalt. Particularly preferred materials are neodymium-iron and samarium-cobalt.

Suitable coatings in accordance with (c)(i) include polypropylene, polyethylene, polystyrene, parylene, titanium nitride, polyimide, chloropolymers, and fluoropolymers, preferably polytetrafluoroethylene (PTFE).

Said at least one permanent magnet performs a fluctuating or oscillating motion, wherein preferably said motion is triggered by a fluctuating or oscillating magnetic field, wherein preferably said magnetic field is generated by an electric current and/or an electromagnet.

Simply speaking, the permanent magnet moves up and down and back and forth, wherein the motion may have regular or repeating components but does not have to, and wherein spatial directions are not particularly limited. Also, the permanent magnet may rotate about one or more axes, usually in addition to translational motion. The permanent magnet may, but does not have to, hit or repeatedly hit a wall of said vessel. The motion of the permanent magnet preferably is not a directed motion. Also, said motion, despite being possibly irregular, generally is about an average position which is located within the mentioned vessel - the permanent magnet does not leave the vessel. The motion of the permanent magnet generally has one or more translational components; and said average position may be somewhere in the middle of said vessel. As such, the motion is different from the motion performed by a magnetic stirrer - which is a rotation, and the average position of the magnet is at or close to the bottom of the vessel containing the liquid to be mixed or stirred.

The term "oscillating" designates a regular motion, whereas the term "fluctuating" is broader and embraces also irregular motion. There is no particular preference in that respect.

The motion of the at least one permanent magnet preferably is triggered by a fluctuating or oscillating magnetic field.

A magnetic field is a common means of controlling position and/or motion of a magnet. Given that in accordance with the invention, the permanent magnet moves, use is made of a fluctuating or oscillating magnetic field in this preferred embodiment.

Preference is given to said magnetic field being generated by an electric current. It is well established that electric and magnetic fields are interrelated; in particular that an electric current generates a magnetic field. As a consequence, controlling the electric current is a means of controlling the magnetic field generated thereby.

Alternatively, or in addition, but less preferred, said magnetic field may be generated or modulated, respectively, by an external permanent magnet. The term "external" means that such magnet is not located within said vessel. When using an external magnet, the magnetic field generated thereby may be rendered fluctuating or oscillating by corresponding movement of said external magnet relative to said at least one permanent magnet inside said vessel.

In a preferred embodiment, said magnetic field is generated by an electromagnet. As used herein, the term "electromagnet" embraces, in its simplest implementation, a piece of an electric conductor through which an electric current is flowing when in use. For better control of the magnetic field or for the purpose of generating stronger magnetic fields, particular implementations of the electromagnet are envisaged which are subject of preferred embodiments disclosed further below.

In a preferred embodiment, said electric current fluctuates or oscillates. This behavior may also be referred to as a generic "wave". The amount of an electric current is known as amperage.

In a preferred embodiment, amperage of said electric current as a function of time is (i) a rectangular function; (ii) a sinusoidal function; (iii) a triangular function; (iv) a sawtooth function; or (v) a combination or convolution of any one of (i) to (iv).

Given that the electric current oscillates or fluctuates, this also applies to patterns (i) to (v), i.e., said rectangular and said triangular functions are in fact repeating rectangular and triangular functions. The term "pattern" designates a series of events where a given basic event is repeated at least once. In a wider sense, repetition does not have to be a precise repetition - the lengths of e.g. rectangles in a time graph may change (which effectively amounts to a change of frequency, preferred frequencies as well as preferred time dependencies of frequencies being specified further below).

All embodiments (i) to (v) are also referred to as "alternating current" herein.

Particularly preferred is said rectangular function (as referred to as rectangular wave or square wave), more specifically the patterns of repeating rectangular functions. The inventors surprisingly found that this pattern triggers particularly vigorous motion of the at least one permanent magnet, wherein such vigorous motion is particularly efficient in terms of mixing.

In said rectangular function, the time intervals of high current and low current (or the current being off) may be the same or different. Means to control the length of said time intervals are known to the skilled person, e.g. those referred to as pulse width modulation (PWM). Of note, the energy transferred to the reaction mixture is not only governed by frequency and amplitude of the electric current, but also by the relative duration of said time intervals.

The energy in a magnetic field per unit volume is defined by *E_{mag}* = ½ *B*²/*µ₀*; for definitions of *B* and *µ*₀ see further below. *E_{mag}* in turn is equal or less than the energy of the electric current which causes the magnetic field. The latter energy can be estimated as *E_{curr}* = *U I t, U* being the voltage and *I* the amperage of the current generating the magnetic field, and *t* is the time during which the current flows. In other words, controlling any one of *B, U, I* and *t* is a means of controlling the amount of energy transferred by the permanent magnet to the contents of said vessel.

Having said that, there are other means of specifying the detailed implementation of the method of the first aspect. This includes specifying one or more of a plurality of parameters which can be more directly controlled or measured. These parameters include frequency and amperage of the current and may furthermore include dimensions of said vessel and a coil, to the extent use is made thereof. Also, the strength of the magnetic field, preferably at the site of the permanent magnet, is a means of quantitatively specifying implementation details. In the following, preferred ranges of the mentioned parameters are specified.

In a preferred embodiment, said electric current fluctuates or oscillates with a given frequency, preferably a frequency of 0.1 Hz to 20 MHz, more preferably 10 Hz to 2 kHz, yet more preferably 50 to 500 Hz or 90 to 300 Hz or 100 to 200 Hz. It is understood that these measures apply not only to sinusoidal current, but to all current profiles specified herein, including, e.g., the repeated rectangular pattern. The term frequency may also apply to fluctuations, i.e., time-dependent behavior which is not regular (such regular time behavior also referred to as "oscillation" herein) and is a means to characterize the timescale of fluctuations. In such a case, the term "frequency" is understood as referring to the average frequency of the fluctuation.

It turns out that at least for vessels with a volume in the one-digit to two-digit mL range as well as for the wells of a standard 96-well plate, lower frequencies between 80 and 300 Hz work particularly well, whereas significantly higher frequencies such as around 1000 Hz, while inducing vibration of the permanent magnet, might not trigger the full range of motion which covers a significant portion of the volume of the vessel. This does not mean that higher frequencies are not beneficial. Also, they may be used in conjunction with lower frequencies (see below).

More generally speaking, a preferred frequency range is a range which ensures that the permanent magnet not only vibrates or rotates, but performs a translational motion which explores the entire volume or substantially the entire volume of the material to be processed with the method of the invention. Said volume generally is the total volume of the liquid phase as contained in said vessel.

In other words, while guidance is given above with regard to vessels with a volume in the one-digit to two-digit mL range and 96-well plates, the frequency ranges may need adaptation for vessels with significantly smaller volume, significantly larger volume, or special geometries. To give an example, it is expected that for smaller volumes such as the wells of high-density microtiter plates (e.g. 1536-well plates) higher frequencies, e.g. of about 1 kHz such as above 200 Hz, lead to a motion of the permanent magnet which is comparable to the motion seen in larger vessels at lower frequencies. In any case, a skilled person provided with the guidance given in this specification, can explore and optimize in a straightforward manner the parameters controlling motion of said at least one permanent magnet. As explained further above, preference is given to the permanent magnet performing translational motion, preferably in addition to rotation.

In a preferred embodiment, said frequency is kept constant throughout while said method is performed.

In an alternative preferred embodiment, said frequency changes as a function of time.

In a further preferred embodiment, more than one frequency is applied at a given point in time. In such a case, each frequency of such a plurality of frequencies may be chosen from any of the preferred intervals given above. Particularly preferred in case of two frequencies is that the first frequency is between 50 Hz and 500 Hz and the second frequency between 80 Hz and 20 MHz. In other words, this preferred embodiment provides for the superposition of a plurality of frequencies.

More than one frequency includes 2, 3, 4, 5, 6, 7, 8, 9 and 10 different frequencies. Such plurality of frequencies may be applied throughout in place of a single frequency - which means that they are applied during the entire performance of the method. Also, a plurality of frequencies, or different pluralities of frequencies may be applied in different time intervals within a longer time span. Within said longer time span, and in addition to time intervals where more than one frequency is applied, there may be one or more, such as 2, 3, 4, 5, 6, 7, 8, 9, or 10 time intervals where only one frequency is applied.

In a further preferred embodiment, said frequency is, and, in case more than one frequency is applied, the frequencies are not constant over time, and preferably is/are switched or gradually changed between two or more frequencies, preferably in a periodic manner.

Exemplary regimes are (120 Hz -1000 Hz)ₙ, (200 Hz -1000 Hz)ₙ, or (100 Hz - 800 Hz)ₙ, wherein n is an integer, e.g. between 2 and 1000, such as between 10 and 100, and specifies the number of times the frequency pattern in brackets is to be repeated.

The duration of the time interval with constant frequency and / or constant amperage is not particularly limited. Envisaged are time intervals between 1 sec and 1 day, such as between 1 min and 1 hr.

In a further preferred embodiment, said electric current (a) has an amperage I between 20 mA and 100 A, preferably between 0,1 and 20 A; (b) exposes said magnet to a magnetic field strength between 0,02 and 10⁹ A/m, preferably between 10 and 10⁶ A/m; and/or (c) is applied for a time span *t* between 1 sec and 1 week, such as between 10 min and 5 hrs.

The magnetic field strength *H* determines the intensity of the field and is measured in A per meter. *H* has to be distinguished from the magnetic flux density B which is particularly relevant in setting where a core is used to re-enforce the magnetic field of an electric current.

Keeping in mind that, as disclosed above, the amperage as a function of time fluctuates on a timescale governed by the frequencies disclosed herein, there is no constant amperage on the timescale of the fluctuation. Yet, for practical purposes, and in line with established practice in electrodynamics, an alternating current may be quantified in terms of its average amperage. The above values are average amperages in that sense. Of note, the average is preferably over the time scale of the fluctuations. That means, to the extent intermittent current is used, there will be an average amperage, preferably within the ranges specified above, when the current is on, and there will be zero amperage when the current is off.

In a preferred embodiment, the amplitude of fluctuation or oscillation is (a) constant; or (b) changes over time, preferably on a timescale which is slower than the timescale of said fluctuation or oscillation.

This embodiment refers to the amplitude of motion of said electric current. The amplitude of oscillation or fluctuation of an electric current is governed by the amperage.

In a further preferred embodiment, said current is intermittent and/or said amperage changes over time, preferably in a periodic manner. This change over time is generally on a time scale which is slower than the time scale defined by the frequency of the alternating current. In other words, if an alternating current changes over time in the sense of this embodiment, the time dependency of the current is a superposition of two patterns or waves: a generally fast fluctuation which is inherent to an alternating current, and a generally slower change.

An exemplary intermittent pattern is a repetition of the sequence on (1 min) - off (1 min). Other preferred time intervals are given above. Advantages of intermittent patterns allow for keeping temperature constant or substantially constant, especially if it is observed that the contents of the vessel are heating up.

In a further preferred embodiment, a power of between 0 and 1000 W, preferably between 1 and 200 W, is applied.

In a further preferred embodiment, the electric current is powered by an electric power source. Preferably, the electric power source has an electric potential or voltage *U* in the range between about 0 and 240 V such as between 0.1 and 75 V. These values refer to the mean voltage applied.

In a further preferred embodiment, said electromagnet comprises at least one coil, wherein preferably said coil (a) has a plurality of windings, such as between 1 and 10⁴, preferably between 10 and 1000; and/or (b) comprises at least one Helmholtz coil; and/or (c) comprises at least one core.

Exemplary numbers of a plurality of coils are 2, 3, 4, 5, 6, 7, 8, 9, 10, 20, 50, 100, 150, 200, 300, 400 and 500.

The term "Helmholtz coil" is established in the art and refers to an arrangement of typically two identical coils spaced apart such that their axes of rotational symmetry align or coincide. The magnetic field in the space between the coils is particularly homogenous and/or particularly strong. Also, a plurality of Helmholtz coils, such as two Helmholtz coils may be used.

Further arrangements of a plurality of coils are known to provide for extended spatial regions of particularly homogeneous magnetic field. A further example is a Maxwell coil.

A core serves to re-enforce the effect of the magnetic field. A core is preferably made of ferromagnetic material such as iron, in particular soft iron. The magnetic flux density *B* is related to magnetic field strength as follows: *B* = *µᵣµ₀ H. µ₀* is the magnetic permeability of vacuum and as such a fundamental physical constant. *µᵣ* on the other hand is the relative permeability and determines the degree of re-enforcement of the magnetic flux density by a given material, e.g. a ferromagnetic core when under the influence of a magnetic field. Typically, *µ*ᵣ for ferromagnetic materials to be used as a core is between 103 and 10⁶ such as between 200000 and 400000, e.g. about 300000. Suitable core materials comprise powdered metals, laminated metals, annealed metals such as annealed iron, ceramics, and solid metals.

Preferred values of *B* in the absence of a core are between 10⁻⁸ and 10⁴ Tesla (T), such as between 10⁻⁵ and 1 T. If a core with a specific relative permeability is used, the values of B are to be multiplied with said relative permeability. As such, preferred values of B in the presence of a core are between 10⁻² and 10⁹ such as between 1 and 10⁶ T. Generally, these values refer to *B* at the site of the permanent magnet or within said vessel.

In terms of geometry, preferred coils are circular. Preferred diameters are between 1 mm and 1 m or 1 mm and 0,5 m, such as between 2 mm and 300 mm or 2 mm and 200 mm. Envisaged are also different geometries such as coils with a square, a rectangular or a triangular shape (i.e., all or part of the windings are a square, a rectangle or a triangle). Finally, and noting that a coil is not an indispensable requirement for an electromagnet, also an arrangement of two antiparallel wires may be used ("antiparallel" referring to the direction of the electric current flowing through the two wires at a given point in time).

In a further preferred embodiment, more than one coil is used, preferably between 2 and 10⁴ such 2,3,4,5, 6, 7,8, 9, or 10 coils, or between 10 and 1000 coils. It is understood that each coil may have one or a plurality of windings, preferred numbers of windings being disclosed herein above.

In a preferred embodiment, said magnetic particles carry at least one moiety on their surface, wherein said moiety is selected from (i) a moiety capable of binding a target molecule, said moiety preferably being a binding protein, affinity chromatography material, absorbing material, adsorbing material, a probe, or a primer; (ii) a moiety capable of converting at least one starting molecule into at least one product molecule, said moiety preferably being an enzyme or chemical catalyst; and (iii) a moiety capable of forming an adduct with a target molecule; wherein said target molecule or starting molecule, respectively, is present or suspected to be present in said liquid phase.

Magnetic particles, both functionalized and non-functionalized, are available from a number of manufacturers, e.g. Resyn Biosciences (Pty) Ltd., Thermo Fisher Scientific, Creative Diagnostics, Nanopartz Inc., Alpha Nanotech Inc., Spherotech Inc., and Stratech. Also, the skilled person can prepare functionalized magnetic particles using known procedures,_see, e.g. Sun et al., Current pharmaceutical biotechnology, 10 753-60 (2009) and Pérez-Ruiz et al., New Biotechnology 33, 755-762 (2016).

Preparation of magnetic particles as such is described, e.g. in Shang et al., Langmuir, 22, 2516-2522 (2006), Oberacker et al., Bio-protocol 9(20): e3394, and Paulke BR., Buske N., Winoto-Morbach S. (1997) Synthesis Studies on Paramagnetic Polystyrene Latex Particles. In: Häfeli U., Schütt W., Teller J., Zborowski M. (eds) Scientific and Clinical Applications of Magnetic Carriers. Springer, Boston, MA.

Said target molecule or said starting molecule will generally be dissolved or suspended in said liquid phase.

An exemplary binding moiety in accordance with item (i) of the above preferred embodiment is ZrO₂. ZrO₂ binds to phosphopeptides. Example 2 illustrates a use in accordance with the invention of magnetic particles with ZrO₂ moieties.

Examples of enzymes include trypsin as well as other proteases such as LysC, GluC, AspN, ArgC or chymotrypsin which cleave proteins, polypeptides and larger peptides into fragments which, owing to their size, are particularly amenable to downstream analytical methods such as mass spectrometry (MS). Example 1 as included herewith demonstrates the use of trypsin-coated magnetic particles in the context of both an implementation of the present invention and an art-established method of magnetic particle handling.

In a further preferred embodiment, said method of the first aspect further comprises or further consists of: (c) removing, or rendering static, and/or rendering said magnetic field incapable of triggering said motion; and allowing said magnetic particles to gather on said permanent magnet; and (d) removing said liquid phase.

The words "further consists of" as herein refer to those implementations where the method consists of a closed number of steps. In case of the embodiment above, these are steps (a) and (b) as defined in accordance with the first aspect and steps (c) and (d) which are subject of said embodiment.

The removal of the magnetic field may be achieved by physically moving the external magnet away from the vessel to an extent that the magnetic field generated by the external magnet becomes negligible at any location within the vessel. Alternatively, or in addition, and this applies to those implementations of the external magnet which are electromagnets, the electric current flowing through the electromagnet may be significantly reduced or turned off.

In a yet further alternative, said magnetic field is not removed or not removed to an extent which would be sufficient to stop the mixing/ dispersion process. In that case, the magnetic field is changed in such a manner that it no longer triggers said fluctuating or oscillating motion of said permanent magnet. As disclosed further below, said magnetic field, in order to trigger such type of motion is generally fluctuating or oscillating in nature as well. By changing such oscillating or fluctuating magnetic field to a static one, the motion of said permanent magnet will cease. This is sufficient to stop the mixing process. Generally speaking, any modification of said magnetic field which renders it incapable of triggering said motion is suitable to stop the mixing process.

Step (d) requires removal of the liquid phase. Of note, said liquid phase at this stage might not be exactly the same liquid phase which was in the vessel prior to adding the magnetic particles. For example, if said liquid phase contained at the beginning a compound or an analyte which binds to the moieties on the surface of the particles, said compound or analyte will be present in lower amounts or absent from the liquid phase to be removed in step (d).

Generally, the gathering phase takes a few seconds such as from 0.1 to 600 sec, 1 to 60 sec, or 10 to 30 sec.

According to this preferred embodiment, said at least one permanent magnet is not only a means of dispersing said magnetic particles, but also serves to collect said particles once the magnetic oscillation or fluctuation is absent or turned off. While also many prior art approaches use a magnet for collecting magnetic particles, said magnet is generally external to said vessel and furthermore requires a device which moves said magnet either into the proximity of the vessel or away therefrom. Another disadvantage of such prior art methods is that any vessel, at least to some extent, will shield the magnetic field such that theoretically possible magnetic flux densities are actually not achieved inside the vessel. Therefore, most prior art technologies need specially designed magnets with very high magnetic field strengths.

The present invention is different from these approaches in that said at least one permanent magnet is located inside the vessel holding the sample, reaction mixture or mixture to be purified.

The above reviewed Chemagic method does immerse a non-permanent, magnetizable rod into the mixture which by means of electromagnetism exerts a magnetic field within said mixture. Yet, the present invention is also distinguished therefrom: the at least one permanent magnet used by the present invention is not integrated into a device which mechanically moves a magnetic rod, but it is immersed in the reaction mixture held by the vessel. Owing to this difference, the permanent magnet used in this invention becomes a disposable element. The risk of cross-contamination introduced by the mentioned magnetic rod is avoided.

While the method of the first aspect has been disclosed above by referring to at least one permanent magnet, it is of note that any magnetic body may be employed for that purpose. The term "magnetic body" as used herein refers to an assembly of particles at least one of which is a magnet and assembling of said particles is mediated by magnetic fields of said at least one magnet. In brief, in a magnetic field, the obtained assembly essentially behaves like a single magnet; for details see further below. In terms of size, said particles will be larger than the molecules to be fragmented (and smaller than the vessel or reactor where the method is performed).

In a second aspect, the present invention provides a method of separating a compound from a mixture, said method comprising or consisting of: (a) in a vessel, bringing said mixture into contact with magnetic particles and at least one permanent magnet, wherein said magnetic particles carry a moiety which is capable of binding said compound; and (b) triggering a fluctuating or oscillating motion of said permanent magnet using a magnetic field; thereby separating said compound from the remainder of constituents of said mixture.

As noted above, said fluctuating or oscillating motion of said permanent magnet is a means of mixing: it brings said binding moiety into contact with said compound, allows the binding to occur which in turn provides the basis for separation from the remainder of the constituents of the liquid phase.

This aspect exploits the advantages of the method of the first aspect while making use of a particular type of functionalized particles, namely, magnetic particles with a binding moiety. As noted above, "binding" as used herein refers to non-covalent interactions between the moieties on the particles and the compound at issue. Such particles are generally designed for separation processes which may be analytic or preparative. As a consequence, the liquid phase of the first aspect is here implemented as a mixture: in addition to the compound of interest (also referred to as "analyte" in some instances), such mixture may comprise other constituents which are not of interest or undesirable (such as contaminants), and the method of the second aspect permits to remove said other constituents such as contaminants and obtain the compound of interest in enriched or pure form. Generally speaking, the method of the second aspect embraces enrichment methods, depletion methods, purification methods, isolation methods, separation methods, fractionation methods, and clean-up procedures.

Preferred embodiments of the binding moiety include those which are disclosed above as preferred embodiments of the method of the first aspect. These preferred embodiments of the method of the first aspect define preferred embodiments of all aspects of this invention. Further preferred or exemplary moieties are disclosed further below, and in the Examples enclosed herewith.

A key distinction from many prior art methods is that the magnet controlling movement and location of the magnetic particles is not external to the vessel but present within the reaction mixture. Thereby, two functions are provided by a single element: magnetic particle handling and thorough mixing of the reaction mixture.

Also deviant from the art-established methods, the magnet can generally be a disposable element. Carry-over between samples by the magnet is thereby easily avoided.

While the known devices for magnetic separation require a mechanical element for moving the magnet(s), this is rendered dispensable by using an electromagnet as external magnet: controlling the current flowing through the electromagnet triggers the desired motion of the permanent magnet within said vessel.

In a preferred embodiment, said method further comprises or further consists of: (c) removing, rendering static, and/or rendering said magnetic field incapable of triggering said motion; and allowing said magnetic particles to gather on said permanent magnet; and (d) removing the remainder of constituents of said mixture from said vessel.

As stated above, and this applies to all embodiments relating to the removal of a magnetic field, such removal may be effected by physical motion between external magnet and vessel and/or by reducing or turning off the fluctuating or oscillating electric current flowing through an electromagnet. Furthermore, the magnetic field may not be removed but rendered incapable of triggering motion of the permanent magnet(s) inside the vessel, e.g. by rendering it static.

Removing of the remainder of constituents as well as any other removal of liquid from the vessel (such as removal of used-up washing solution as disclosed below or the removal of eluate, also disclosed below) can be by any art-established means and methods such as, e.g., pipetting.

Gathering of particles on the magnet generally takes place on a timescale to be measured in seconds. A corresponding preferred embodiment of the first aspect applies mutatis mutandis. Having said that, gathering times may depend to some extent on the settings chosen, e.g. on the magnetic field strength of the permanent magnet inside the vessel and the size of the magnetic particles. As will become apparent further below, preference is given to strongly magnetic materials. Also, the permanent magnet is preferably not coated or, if it is coated, the coating does not exert a strong shielding effect on the magnetic field generated by the permanent magnet.

In a preferred embodiment, said method further comprises or further consists of: (e) adding a washing solution to said vessel; (f) triggering a fluctuating or oscillating motion of said permanent magnet using said magnetic field; (g) removing, rendering static, and/or rendering said magnetic field incapable of triggering said motion; and allowing said magnetic particles to gather on said permanent magnet; and (h) removing said washing solution from said vessel; wherein steps (e) to (h) may be repeated.

This embodiment relates to a washing procedure which is adapted to the magnetic particles handling in accordance with the present invention. Accordingly, it makes use of the permanent magnet inside the vessel and the external magnet. The same applies mutatis mutandis to the preferred embodiment below which defines an optional elution procedure which eventually delivers the compound of interest in the eluate.

To the extent repeated washing is performed, this can be done with the same or a different washing liquid. Appropriate choices can be done by the skilled person depending on the specific application chosen.

In a preferred embodiment, said method further comprises or further consists of: (i) adding an eluent to said vessel, wherein said eluent reduces or abolishes binding of said compound to said moiety; (j) triggering a fluctuating or oscillating motion of said permanent magnet using said magnetic field; and (k) removing, rendering static, and/or rendering said magnetic field incapable of triggering said motion; and allowing said magnetic particles to gather on said permanent magnet.

In a preferred embodiment, said method further comprises or further consists of: (1) separating the obtained eluate from said magnetic particles and said permanent magnet.

In other words, an exemplary enrichment or clean-up process introduces the magnetic particles to the sample containing molecules or material of interest. The contact to the magnetic beads introduces a binding reaction. Adding a permanent magnet to the sample/magnetic particle dispersion introduces magnetic separation and collection of said magnetic particles on the magnet. The remaining liquid, now devoid of target molecules is then simply removed and a washing solution is added. Subsequently a fluctuating or oscillating field is introduced to disperse and efficiently wash the magnetic particles. The field is then turned off to re-collect the particles and remove the now consumed washing solution. Finally, an elution buffer is added to the magnetic particles and the fluctuating or oscillating magnetic field is re-introduced to perform efficient elution. After the fluctuating or oscillating magnetic field has been turned off again, the eluate containing purified molecules of interest can be further processed or analyzed.

In a preferred embodiment, said method further comprises or further consists of one or both steps of (a0) fragmenting said compound; and (a00) lysis of cells, to the extent present, comprising said compound; wherein step (a00), if performed, is to be effected prior to steps (a0) and (a); and step (a0), if performed, is to be effected prior to step (a).

This preferred embodiment relates typically to those cases where the mixture or liquid phase to start with is more complex. "Complexity" may refer to the analytes under consideration - they may be large macromolecules, e.g. of biological origin - and/or to the matrix into which the analytes are embedded, for example they may be located within or on the surface of biological cells or tissues.

Depending on the nature of the sample, step (a0) or both steps (a00) and (a0) may be of interest.

In a particularly preferred embodiment, step (a00) and/or step (a0) are effected by triggering a fluctuating or oscillating motion of said permanent magnet by said external magnet.

Depending on any downstream analysis that may be of interest, fragmenting of large macromolecules to give rise to smaller compounds may be desirable. This applies to most mass spectrometry sample measurements. As described in applicant's earlier patent applications EP 20174469.5 and EP 20174484.4, a moving magnet is a useful means of fragmenting biological macromolecules such as proteins or nucleic acids. This is the subject-matter of above disclosed step (a0). The same applies mutatis mutandis to those instances where a macromolecule is not the molecule of interest, but inactivation thereof is desirable.

Analogously, and as described in applicant's earlier patent applications WO 2020/002577 and EP 20174484.4, a moving magnet is also a means of breaking up biological cells and thereby making their contents accessible. This is the subject-matter of above disclosed step (a00).

In a third aspect, the present invention provides a method of producing at least one product molecule, said method comprising or consisting of: (a) in a vessel, bringing at least one starting molecule into contact with magnetic particles and at least one permanent magnet, wherein said magnetic particles carry a moiety which is capable of converting said at least one starting molecule into at least one product molecule; and (b) triggering a fluctuating or oscillating motion of said permanent magnet using a magnetic field; thereby obtaining said at least one product molecule.

The method of the third aspect makes use of functionalized magnetic particles which carry a catalyst on their surface. As stated further above, such catalyst may be an enzyme or a chemical catalyst. The catalyzed reaction is not particularly limited. Examples include cleavage such as hydrolysis, and synthesis reactions such as polymerization.

In a preferred embodiment, said at least one product molecule does not remain bound to said particles. In other words, once the catalyzed reaction is completed, the product can freely diffuse away from the magnetic particle.

In a further preferred embodiment, said method further comprises or further consists of: (c) removing, rendering static, and/or rendering said magnetic field incapable of triggering said motion; and allowing said magnetic particles to gather on said permanent magnet; and (d) removing said at least one product molecule from said vessel.

In a fourth aspect, the present invention provides a method of derivatizing a first compound, said method comprising or consisting of: (a) in a vessel, bringing said first compound into contact with magnetic particles and at least one permanent magnet, wherein said magnetic particles carry a moiety which is capable of forming an adduct with said first compound; and (b) triggering a fluctuating or oscillating motion of said permanent magnet using a magnetic field; thereby obtaining said adduct.

The method of the fourth aspect makes use of magnetic particles which carry a chemically reactive group on their surface, wherein the chemical reaction occurring with said first compound and said reactive group leads to the formation of a covalent bond connecting the two.

Reactive groups are not particularly limited and can be chosen by the skilled person without further ado depending on the envisaged application. For example, if the first compound contains a primary amino group, a suitable moiety is an NHS ester.

The recited adduct may also be referred to as conjugate. The adduct comprises or consists of said compound and said moiety, wherein the two are connected by at least one covalent bond.

Preferably, said adduct remains bound to said particles.

It is understood that such adduct formation is also a means of producing functionalized magnetic particles. In other words, the method of the fourth aspect may be used as a method of producing functionalized magnetic particles.

Moreover, functionalized magnetic particles obtained via that route may be used as magnetic particles in the context of any of the aspects of this invention.

Preferably, said method further comprises or further consists of: (c) removing, rendering static, and/or rendering said magnetic field incapable of triggering said motion; and allowing said magnetic particles to gather on said permanent magnet; and (d) removing material which is not bound to said particles from said vessel.

In a preferred embodiment, said method further comprises or further consists of: (e) adding a washing solution to said vessel; (f) triggering a fluctuating or oscillating motion of said permanent magnet using said magnetic field; (g) removing, rendering static, and/or rendering said magnetic field incapable of triggering said motion; and allowing said magnetic particles to gather on said permanent magnet; and (h) removing said washing solution from said vessel; wherein steps (e) to (h) may be repeated.

As is common in the art, washing steps in analytical or preparative procedures may, but do not have to be repeated, for example, 2, 3, 4, 5, 6, 7, 8, 9, or 10 times. This applies to all aspects of this invention.

Related to the fourth aspect, the present invention, in a fifth aspect, provides a method of solid-phase synthesis, said method comprising performing the method of the fourth aspect, or any of the preferred embodiments thereof, once or repeatedly, wherein said magnetic particles constitute the solid phase.

The number of repetitions is not limited. It depends on the application chosen, the desired properties (such as length) of the product, and, as common in synthetic procedures, on the yield of each repetition and the desired yield of the final product.

In a preferred embodiment of the method of the fifth aspect, during a second and a further performance of said method of the fourth aspect, second and further compounds, respectively, are added to said vessel, whereby a product is obtained which comprises said first, second and further compounds as building blocks.

This preferred embodiment can be implemented as a progressive chain elongation reaction, wherein in the first round an adduct is formed between the above described reactive moiety on the magnetic particles and the first compound, and in a second round a conjugate is formed between said adduct and a second compound.

This can be achieved, for example, by using a first compound which has, in addition to a group which reacts with the moiety on the magnetic particle, a further group which, either as it is or upon suitable activation, is capable of forming a covalent bond with said second compound. This also applies mutatis mutandis to further rounds, to the extent they are to be performed when working the method of the fifth aspect.

The obtained product may but does not have to have a sequential structure of the general type first compound - second compound - third compound - etc. Depending on the compounds and/or the moiety on the magnetic particle, also branched or crosslinked products may be obtained.

In order to avoid unwanted side reactions, suitable protection groups may be employed. Removal of the protection group would take place when a reaction of the protected group shall occur. Repeated cycles of protection and deprotection are art-established, e.g. for the purpose of solid phase peptide synthesis, and make use of protection groups such as fMoc and Boc.

In a preferred embodiment, said adduct or said compound, respectively, is cleaved off said magnetic particles, e.g. by acidic, basic or light induced cleavage of a covalent bond.

This is a commonly used measure in solid phase synthesis: once the desired product is obtained, it is still bound to the solid phase, i.e., the magnetic particles. In order to handle or process the product further, it is cleaved from the solid phase.

The use of the methods of the invention for synthetic purposes is not limited to the above described method of solid phase synthesis where the first building block is covalently bound to the particle (at least until the point in time where the final product is to be cleaved off). Indeed, repeated rounds of synthesis may be performed by employing non-covalent interactions. Examples thereof include hybridization between at least two nucleic acid species as they occur, e.g., in the course of a polymerase chain reaction (PCR).

It is understood that in the course of any such method involving multiple subsequent reactions, further steps in addition to those which are explicitly recited above may be beneficial or necessary. Such further steps can be applied by the skilled person without further ado and depending on the specific synthesis or other method to be performed. Examples of such additional steps include the addition of further agents in addition to the compounds which eventually form the building blocks of the synthesized molecule. Such further agents include activators, blocking agents, neutralizing or pH-shifting agents and the like. Exemplary blocking agents are fMoc and Boc as mentioned above.

In a preferred embodiment of the method of any one of the above disclosed aspects, at least one ferromagnetic particle or bead is added together with said at least one permanent magnet. This is a preferred embodiment of the methods of all aspects of this invention. The inventors surprisingly found that adding a ferromagnetic particle or bead, preferably of a size which is comparable or identical to the size of the permanent magnet, improves mixing by rendering motion of the permanent magnet and said bead more vigorous. In such a setting, a plurality of permanent magnets and/or a plurality of ferromagnetic beads may be used, even though preference is given to exactly one permanent magnet and exactly one ferromagnetic bead.

In a preferred embodiment of the methods of all aspects, one, more, or all of the respective steps (d), (e), (h), (i) and (l are effected by pipetting.

Generally speaking, and especially in the context of such pipetting, a yet further magnet may be employed. To explain further, during the phase where the magnetic field does not trigger a fluctuating or oscillating motion of the permanent magnet(s) inside the vessel and the magnetic particles are allowed to gather on the surface of said permanent magnet(s), they will generally end up at the bottom of the vessel owing to gravity. This in turn may render pipetting less effective or more cumbersome. Therefore, said yet further magnet, e.g. another permanent magnet may be brought into the vicinity of an external wall of said vessel. This provides for the permanent magnet(s) inside the vessel as well as the magnetic particles to be located at said wall (inside the vessel). When pipetting at this point, the pipette tip may be inserted into the vessel and down to the very bottom thereof.

In a preferred embodiment of the methods of all aspects, said magnetic field is generated by an external magnet, wherein said external magnet preferably is (1) an electromagnet, wherein a fluctuating or oscillating electric current flows through said electromagnet; and/or (2) a further permanent magnet or an electromagnet, wherein said vessel and said further permanent magnet or said electromagnet are moved relative to each other in a fluctuating or oscillating manner.

Particularly preferred is option (1).

In a preferred embodiment of the method of any one of the above disclosed aspects, said magnetic particles are paramagnetic, ferrimagnetic or ferromagnetic. A preferred material is magnetite. The magnetic particles do not have to be permanently magnetic. Yet, the materials which are suitable materials for the at least one permanent magnet are also suitable magnetic materials for said magnetic particles.

The method of any one of the preceding claims, wherein said analyte or compound is a nucleic acid, a ribonucleic acid, a sugar, a saccharide, a protein, a polypeptide, a peptide, and/or a lipid.

In a preferred embodiment of the method of any one of the preceding aspects, different steps are effected at different temperatures. Such regimes are useful for polymerase chain reactions (PCR), enzymatic processes which employ enzymes with a temperature optimum above ambient temperature, or where elevated temperature is a means of interfering with binding such as decreasing binding.

The method of any one of the preceding claims, wherein a plurality of vessels, for example the vessels of a microtiter plate, are processed simultaneously.

Related to all previous aspects, the present invention furthermore relates to the use of at least one permanent magnet, magnetic particles, and a magnetic field for mixing a reaction mixture in a vessel, wherein said at least one permanent magnet and said particles are in said vessel, and wherein preferably said magnetic field is generated by a further magnet which is outside said vessel.

As described in relation to the methods of the invention, said further magnet may be implemented as a permanent magnet or an electromagnet such as a coil.

In a further aspect, the present invention provides elements suitable for performing the methods and uses of the invention as a kit. Such a kit comprises or consists of (i) magnetic particles; (ii) at least one permanent magnet; and (iii) at least one liquid reagent such as a buffer.

In a preferred embodiment, said magnetic particles are functionalized, preferably with binding moieties and/or reactive moieties.

Generally speaking, preferred embodiments of the methods of the invention also define preferred embodiments of the use, the kit, and the device of the invention. For example, in a preferred embodiment, said kit comprises, in addition to said at least one permanent magnet, at least one ferromagnetic bead or a non-magnetic bead as described in more detail in relation to the method of the invention.

In a further preferred embodiment, said kit further comprises or further consists of (iv) a manual comprising instructions for performing the method of any one of the first to the fifth aspect.

In a further preferred embodiment, the kit further comprises of further consists of at least one vessel or at least one array of vessels, said vessel or array of vessels preferably being as defined herein above.

In a further aspect, the invention provides a device comprising or consisting of (i) means for generating a magnetic field; and (ii) a control unit which is configured to modify said field over time in order to perform the method of any of the aspects of the present invention.

To explain further, and considering for example steps (b) and (c) of the method of the first aspect, said control unit causes the magnetic field to trigger a fluctuating or oscillating motion of said permanent magnet for the duration of step (b), and renders the magnetic field incapable of triggering such motion during step (c), for example by turning the field off.

Said means for generating a magnetic field are preferably those defined further above in relation to the methods of the invention, i.e., an electrical conductor or a permanent magnet.

The electromagnet may be an electric conductor, preferably at least one coil, more preferably a Helmholtz coil. The opening of said coil is configured to accommodate a vessel. Said vessel preferably is as defined further above, e.g. it may be a single vessel or an array of vessels.

When using an electromagnet, said control unit is preferably configured to deliver any of the preferred time profiles of electric current as described in detail in relation to the methods of this invention.

In a preferred embodiment, said device further comprises or further consists of said vessel.

In a further preferred embodiment, said device further comprises or further consists of at least one permanent magnet and/or magnetic particles, wherein, to the extent the device comprises said vessel, said permanent magnet and/or said particles are preferably located inside said vessel.

The permanent magnet and the magnetic particles are defined further above.

Generally speaking, preferred embodiments of one aspect define, mutatis mutandis, preferred embodiments of another aspect.

It is understood that said device is configured such that said particles and said magnet are inside said vessel and exposed to the magnetic field, said field preferably being generated by said electromagnet or said further permanent magnet outside said vessel.

The term "accommodate" means that said coil has an opening wide enough such that said vessel fits inside said opening, the consequence being that the contents of said vessel is located where the magnetic field generated by said coil when in use is particularly strong and/or particularly homogeneous. Preferably, and in case of said vessel having a circular cross section (such as in case of a cylindrical vessel), the inner diameter of said coil (a circular coil in that case) is only slightly wider than the outer diameter of said vessel. "Slightly wider" may mean between 0.01 and 10% such as between 0.1 and 1% wider.

In case of an array of vessels (such as a microtiter plate), the coil may be such that it is just slightly wider than said array. If said array is rectangular in shape, a coil such as a Helmholtz coil with a rectangular shape may be employed.

The control unit may further comprise a power source or an adapter to be connected to an electric plug.

The device may be provided together with a manual comprising instructions for performing any of the methods of this invention.

The Figures show:

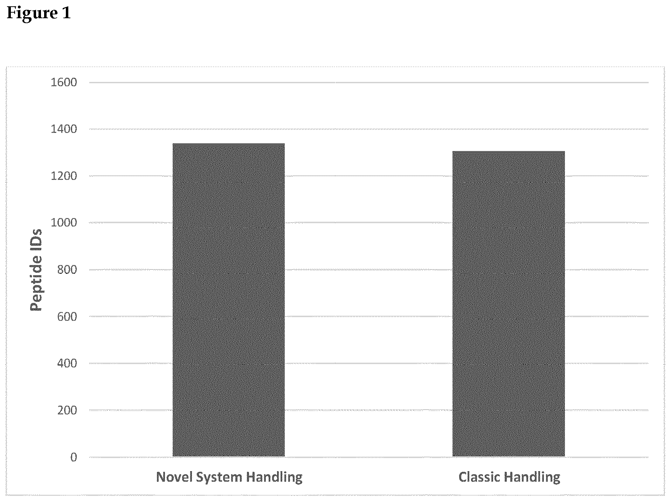

**Figure 1****:** "Novel System Handling" shows peptide identification by means of MS when handling magnetic particles during the preceding sample preparation in accordance with the present invention. In comparison, classic handling shows the number of distinct identified peptides when using an art-established magnetic separator.

The Examples illustrate the invention.

### Example 1

### Performance comparison with prior art magnetic separator

### Material

*Saccharomyces cerevisiae* cell pellets with approximately 100 µg protein content were used for digestion tests. A permanent Neodymium magnet was used for beads handling (MagnetExpert; spheric, 2mm). For lysis and clean-up, the iST-Kit including buffers and plastic ware was used (PreOmics GmbH, P.O.00001). Trypsin magnetic microparticles were provided by ReSyn Biosciences (ReSyn Biosciences (Pty) Ltd). A Helmholtz-coil setup was used to generate an external, oscillating magnetic field.

### Methods

### Classical magnetic particle handling

*S. cerevisiae* cell lysis was prepared as described in Standard iST sample preparation (PreOmics GmbH, iST-Kit, P.O.00001). 750 µg immobilized trypsin beads (Trypsin magnetic microparticles) were equilibrated with 2x 70 % ethanol; 1x 1 % ammoniumhydroxide 3x 50 mM Tris, pH 8. For each equilibration step, beads were incubated at 500 rpm for 3 min and supernatant was removed using a magnetic separator and waiting for 30s for the magnetic microparticles to collect. Equilibrated beads were mixed with denatured yeast samples. Digestion was performed at 37 °C and 500 rpm for 1 h. Prior to peptide purification and LC-MS analysis (see standard iST sample preparation), magnetic trypsin beads were removed from samples using the magnetic separator and 100 µl of stop buffer were added. After elution, purified peptides were dried in the SpeedVac and resuspended in 2% acetonitrile, 0.1% trifluoroacetic acid. Samples were analyzed on a ThermoFisher Scientific Easy n-LC 1200 system coupled with a Thermo LTQ Orbitrap XL. Peptide loads of 5 µg were separated on a home-made C18 column applying a 45 min gradient and tandem mass spectrometry was performed using a DDA Top 10 method. The MS/MS data was searched against a yeast database using the MaxQuant software with default settings except using unspecific search.

### Novel system magnetic particle handling

*S. cerevisiae* cell lysis was prepared as described in Standard iST sample preparation (PreOmics GmbH, iST-Kit, P.O.00001). 750 µg immobilized trypsin beads (Trypsin magnetic microparticles) were equilibrated with 2x 70 % ethanol, 1x 1% ammonium hydroxide and 3x 50 mM Tris, pH 8 on the Helmholtz coil setup with a 2 mm round Neodymium magnet. For each equilibration step, samples were incubated on the Helmholtz system for 3 min applying a square wave function at 120 Hz and supernatant was removed after turning the field off and letting the magnetic particles collect for 10s. Equilibrated beads were mixed with denatured yeast samples and digestion was performed on the Helmholtz system for 60 min applying a square wave function at 120 Hz. Prior to peptide purification and LC-MS analysis (see Standard iST sample preparation), magnets and magnetic trypsin beads were removed from samples and 100 µl of stop buffer were added. After elution, purified peptides were dried in the SpeedVac and resuspended in 2% acetonitrile, 0.1% trifluoroacetic acid. Samples were analyzed on a ThermoFisher Scientific Easy n-LC 1200 system coupled with a Thermo LTQ Orbitrap XL. Peptide loads of 5 µg were separated on a home-made C18 column applying a 45 min gradient and tandem mass spectrometry was performed using a DDA Top 10 method. The MS/MS data was searched against a yeast database using the MaxQuant software with default settings except using unspecific search.

### Results

See Figure 1 and caption thereof.

### Discussion

In terms of the number of peptides identifiable by MS, the method of the invention performs at least as good as the classical handling of magnetic particles. Yet, and as discussed herein above, the method of the invention confers distinct advantages such as there being no requirement for a mechanical movement of the magnet outside the vessel and the avoidance of any cross-contamination.

### Example 2

### Materials

For cell pellets containing approximately 100 µg of yeast proteins, commercially available *Saccharomyces cerevisiae* was resuspended in H₂O and aliquots of 1 ml with OD 0.6 at 600 nm were prepared and centrifuged. Standard sample preparation for LC-MS analysis was performed with the iST kit from PreOmics GmbH (P.O.00001) including buffers, enzymes and plasticware. A permanent Neodymium magnet was used for beads handling (MagnetExpert; spheric, 2mm). ZrO₂ magnetic microparticles were provided by ReSyn Biosciences (ReSyn Biosciences (Pty) Ltd). A Helmholtz-coil setup was used to generate an external, oscillating magnetic field. Peptide clean-up was performed using cation exchange cartridges.

### Method

Sample preparation was carried out according to the PreOmics standard protocol (PreOmics GmbH, P.O.00001) for yeast samples, followed by phosphopeptide enrichment on magnetic microparticles (ReSyn Biosciences), and purification of the sample using the reversed-phase cartridges. Purified peptides were dried to completeness under vacuum at room temperature.

For the enrichment of the phosphopeptides, first the magnetic beads were equilibrated. A spheric Neodymium magnet with a radius of 2 mm was added to 10 µl of bead suspension. The suspension was allowed to clear and the supernatant was taken off. 50 µl of wash buffer (70 % ethanol) was added and the sample mixed by using an external magnetic oscillating field (120 Hz, square wave form, 5 min). After that, the suspension was allowed to clear and the supernatant was taken off. This procedure was repeated once for a total of two washes with this buffer.

50 µl of 1 % ammonium hydroxide was added and the sample was mixed again using the method of the invention (120 Hz, square wave form, 10 min). Again, the sample was allowed to clear, and the supernatant was taken off. The beads were then equilibrated three times using 50 µl of 0.2 M glycolic acid in 5 % trifluoroacetic acid in 80 % acetonitrile using the same procedure as above.

Then 100 µl of 0.2 m glycolic acid in 5 % trifluoroacetic acid in 80 % acetonitrile was added to the dried peptide pellet from above and resuspended properly. The solution was then added to the equilibrated beads and incubated while mixing with the method of the invention (120 Hz, square wave form, 20 min). The sample was allowed to clear and the supernatant was taken off. Unbound sample was removed by adding another 50 µl of 0.2 M glycolic acid in 5 % trifluoroacetic acid in 80 % acetonitrile and mixing with the method of the invention (120 Hz, square wave form, 2 min). The sample was allowed to clear, supernatant was taken off.

100 ul of washing buffer (1 % trifluoroacetic acid in 80 % acetonitrile) was added and the sample was mixed with the method of the invention (120 Hz, square wave form, 2 min) followed by allowing the sample to clear and taking off the supernatant. This step was repeated by another washing buffer (0.2 % trifluoroacetic acid in 10 % acetonitrile).

The phosphopeptides were eluted from the magnetic beads by adding 40 µl elution buffer (1 % ammonium hydroxide) and incubated with mixing using the method of the invention (120 Hz, square wave form, 5 min). The sample was allowed to clear, the supernatant was taken off and transferred to a fresh tube. The elution was repeated twice for a total elution volume of 120 µl.

The tube containing the elution was centrifuged at maximum speed to pellet fragments of the magnetic beads. Using a magnetic separator, the supernatant was taken off and transferred to a new tube. The sample was dried under vacuum at room temperature until completely dry.

Next, a cation-exchange cartridge was equilibrated by adding 200 µl methanol followed by centrifugation in a waste tube (3.800 rcf, 1 min). A second equilibration was performed using 200 µl 2 % acetonitrile, 0.1 % trifluoroacetic acid buffer as described above.

The dried phosphopeptides were resuspended in 200 µl 2 % acetonitrile, 0.1 % trifluoroacetic acid buffer and loaded on the cartridge and centrifuged (3.800 rcf, 1 min). Then 200 µl 0.1 % formic acid was added followed by centrifugation (3.800 rcf, 1 min) for washing the bound peptides. This washing step was repeated twice. The cartridge was transferred to a fresh collection tube, 200 µl of 0.1 % formic acid in 80% acetonitrile was added followed by centrifugation (3.800 rcf, 1 min) for eluting the peptides from the cartridge. The elution step was repeated once for a total of two elution steps. The samples were dried under vacuum at room temperature until completely dry and then resuspended in 6 µl 2 % acetonitrile, 0.1 % trifluoroacetic acid buffer.

Samples were analyzed on a ThermoFisher Scientific Easy n-LC 1200 system coupled with a Thermo LTQ Orbitrap XL. Peptides were separated on a home-made C18 column applying a 45 min gradient and tandem mass spectrometry was performed using a DDA Top 10 method. The MS/MS data was searched against a yeast database using the MaxQuant software with default settings.

### Results

**Discussion**

| Proteins identified | Peptides identified | Phosphopeptides (STY) [%] |
|---|---|---|
| 277 | 419 | 60.632 |

The specificity apparent from the results demonstrates that the method of the invention satisfactorily performs for separation and enrichment purposes.

## Claims

1. A method of dispersing magnetic particles, said method comprising or consisting of:
(a) in a vessel, combining at least one permanent magnet and said magnetic particles in a liquid phase; and
(b) triggering a fluctuating or oscillating motion of said permanent magnet using a magnetic field;
thereby dispersing said particles.

2. The method of claim 1, wherein said magnetic particles carry at least one moiety on their surface, wherein said moiety is selected from
(i) a moiety capable of binding a target molecule, said moiety preferably being a binding protein, affinity chromatography material, absorbing material, adsorbing material, a probe, or a primer;
(ii) a moiety capable of converting at least one starting molecule into at least one product molecule, said moiety preferably being an enzyme or chemical catalyst; and
(iii) a moiety capable of forming an adduct with a target molecule;
wherein said target molecule or starting molecule, respectively, is present or suspected to be present in said liquid phase.

3. The method of claim 1 or 2, further comprising or further consisting of:
(c) removing, rendering static, and/or rendering said magnetic field incapable of triggering said motion; and allowing said magnetic particles to gather on said permanent magnet; and
(d) removing said liquid phase.

4. A method of separating a compound from a mixture, said method comprising or consisting of:
(a) in a vessel, bringing said mixture into contact with magnetic particles and at least one permanent magnet, wherein said magnetic particles carry a moiety which is capable of binding said compound; and
(b) triggering a fluctuating or oscillating motion of said permanent magnet using a magnetic field;
thereby separating said compound from the remainder of constituents of said mixture.

5. The method of claim 4, further comprising or further consisting of:
(c) removing, rendering static, and/or rendering said magnetic field incapable of triggering said motion; and allowing said magnetic particles to gather on said permanent magnet; and
(d) removing the remainder of constituents of said mixture from said vessel.

6. The method of claim 4 or 5, further comprising or further consisting of:
(e) adding a washing solution to said vessel;
(f) triggering a fluctuating or oscillating motion of said permanent magnet using said magnetic field;
(g) removing, rendering static, and/or rendering said magnetic field incapable of triggering said motion; and allowing said magnetic particles to gather on said permanent magnet; and
(h) removing said washing solution from said vessel;
wherein steps (e) to (h) may be repeated.

7. The method of claim 5 or 6, further comprising or further consisting of
(i) adding an eluent to said vessel, wherein said eluent reduces or abolishes binding of said compound to said moiety;
(j) triggering a fluctuating or oscillating motion of said permanent magnet using said magnetic field; and
(k) removing, rendering static, and/or rendering said magnetic field incapable of triggering said motion; and allowing said magnetic particles to gather on said permanent magnet,
wherein preferably said method further comprises or further consists of
(l) separating the obtained eluate from said magnetic particles and said permanent magnet.

8. A method of producing at least one product molecule, said method comprising or consisting of:
(a) in a vessel, bringing at least one starting molecule into contact with magnetic particles and at least one permanent magnet, wherein said magnetic particles carry a moiety which is capable of converting said at least one starting molecule into at least one product molecule; and
(b) triggering a fluctuating or oscillating motion of said permanent magnet using a magnetic field;
thereby obtaining said at least one product molecule.

9. The method of claim 8, further comprising or further consisting of:
(c) removing, rendering static, and/or rendering said magnetic field incapable of triggering said motion; and allowing said magnetic particles to gather on said permanent magnet; and
(d) removing said at least one product molecule from said vessel.

10. A method of derivatizing a first compound, said method comprising or consisting of:
(a) in a vessel, bringing said first compound into contact with magnetic particles and at least one permanent magnet, wherein said magnetic particles carry a moiety which is capable of forming an adduct with said first compound; and
(b) triggering a fluctuating or oscillating motion of said permanent magnet using a magnetic field;
thereby obtaining said adduct.

11. The method of claim 10, further comprising or further consisting of:
(c) removing, rendering static, and/or rendering said magnetic field incapable of triggering said motion; and allowing said magnetic particles to gather on said permanent magnet; and
(d) removing material which is not bound to said particles from said vessel.

12. The method of any one of the preceding claims, wherein said magnetic field is generated by an external magnet, wherein said external magnet preferably is
(1) an electromagnet, wherein a fluctuating or oscillating electric current flows through said electromagnet; and/or
(2) a further permanent magnet or an electromagnet, wherein said vessel and said further permanent magnet or said electromagnet are moved relative to each other in a fluctuating or oscillating manner.

13. Use of at least one permanent magnet, magnetic particles, and a magnetic field for mixing a reaction mixture in a vessel, wherein said at least one permanent magnet and said particles are in said vessel, and wherein preferably said magnetic field is generated by a further magnet which is outside said vessel.

14. A kit comprising or consisting of
(i) magnetic particles;
(ii) at least one permanent magnet; and
(iii) at least one liquid reagent such as a buffer.

15. A device comprising or consisting of
(i) means for generating a magnetic field; and
(ii) a control unit which is configured to modify said field over time in order to perform the method of any one of the aspects of the present invention.
